# FASCICULE DE BREVET EUROPEEN

(11) **EP 2 838 899 B1**
(45) Date de publication et mention de la délivrance du brevet: **05.10.2016**
(21) Numéro de dépôt: 13701119.3
(22) Date de dépôt: 25.01.2013
(51) Int. Cl.: C07D 417/12, C07D 277/34, C07D 417/06, C07D 417/14, C07D 311/66, A61K 31/427, A61P 35/00

(54) **DÉRIVÉS DE THIAZOLIDINEDIONE, LEUR PRÉPARATION ET LEUR UTILISATION DANS LE TRAITEMENT DES CANCERS**
THIAZOLIDINDION-DERIVATE, DEREN HERSTELLUNG UND VERWENDUNG IN DER BEHANDLUNG VON KREBS
THIAZOLIDINEDIONE DERIVATIVES, THEIR PREPARATION AND USE IN THE TREATMENT OF CANCERS

(30) Priorité: 26.01.2012 FR 1250753
(43) Date de publication de la demande: 25.02.2015
(73) Titulaire: Université de Lorraine, 54052 Nancy Cedex (FR); Centre National de la Recherche Scientifique (CNRS), 75794 Paris Cedex 16 (FR)
(72) Inventeur: BOISBRUN, Michel, F-54425 Pulnoy (FR); CHAPLEUR, Yves, F-54000 Nancy (FR); BORDESSA, Andrea, I-22015 Gravedona Ed Uniti (IT); FLAMENT, Stéphane, F-54500 Vandoeuvre-les-nancy (FR); GRILLIER-VUISSOZ, Isabelle, F-54600 Villiers-les-Nancy (FR); KUNTZ, Sandra, F-54740 Crantenoy (FR)
(74) Mandataire: GLN SA
(86) Numéro de dépôt international: PCT/EP2013/051514
(87) Numéro de publication internationale: WO 2013/110796

(56) Documents cités:
- EP-A2- 0 454 501
- WO-A1-2009/105621
- JP-A- 8 286 293
- JP-A- 2007 063 444
- JIH-HWA GUH ET AL: "Development of Novel Adenosine Monophosphate-Activated Protein Kinase Activators", JOURNAL OF MEDICINAL CHEMISTRY, vol. 53, no. 6, 25 mars 2010 (2010-03-25), pages 2552-2561, XP055038069, ISSN: 0022-2623, DOI: 10.1021/jm901773d cité dans la demande
- STÉPHANE SALAMONE ET AL: "Synthesis of new troglitazone derivatives: Anti-proliferative activity in breast cancer cell lines and preliminary toxicological study", EUROPEAN JOURNAL OF MEDICINAL CHEMISTRY, vol. 51, 1 mai 2012 (2012-05-01), pages 206-215, XP055038171, ISSN: 0223-5234, DOI: 10.1016/j.ejmech.2012.02.044
- DASHENG WANG ET AL: "Development of a Novel Class of Glucose Transporter Inhibitors", JOURNAL OF MEDICINAL CHEMISTRY, vol. 55, no. 8, 26 avril 2012 (2012-04-26) , pages 3827-3836, XP055053795, ISSN: 0022-2623, DOI: 10.1021/jm300015m

## Description

### Domaine technique

La présente invention concerne des dérivés de thiazolidinedione, leurs procédés de préparation, et leur utilisation en thérapeutique. Plus particulièrement, la présente invention concerne de nouveaux composés dérivés de thiazolidinedione à usage thérapeutique pour la prévention ou le traitement des cancers, et plus spécifiquement les cancers du sein.

### Etat de la technique

Des dérivés de thiazolidinediones ou de glitazones, tels que la troglitazone (TGZ), la rosiglitazone (RGZ) et la pioglitazone (PGZ) ont été utilisées en thérapeutique humaine pour le traitement du diabète de type 2. Leur activité était décrite comme due à leur propriété activatrice des récepteurs PPARy. Ces récepteurs, dont il existe trois sous-types (α, γ, δ), sont situés dans les noyaux des cellules et sont intimement associés à l'ADN. Leur activation par des composés endogènes (acides gras, prostaglandines) ou synthétiques (molécules de la famille des glitazones ou autres composés plus récents) induit une modulation complexe de l'expression de certains gènes aboutissant à une baisse de la glycémie et de l'insulinorésistance.

Plus tard ont été découvertes les propriétés antiprolifératives de certaines glitazones et en particulier de la TGZ. Cette dernière a même fait l'objet d'essais cliniques de phase II dans le cadre du cancer du sein. La ou les voies par lesquelles la troglitazone exerce son activité antiproliférative sont encore débattues, mais de plus en plus d'études suggèrent qu'elles seraient au moins partiellement PPARγ-indépendantes.

Il est fait mention que les cellules saines ne semblent pas réagir à ces composés de la même façon que les cellules cancéreuses, ce qui est en faveur de leur utilisation en clinique.

Un point négatif a longtemps empêché l'utilisation de la TGZ pour ses propriétés anticancéreuses : cette molécule induit des troubles hépatiques importants de manière non prévisible chez certaines personnes. Ceci n'est pas le cas chez les rongeurs et c'est ainsi que la molécule a pu passer le filtre des essais précliniques. Cette différence entre animaux et humains semble due à des différences de métabolisation et les développements ultérieurs de molécules semblables se feront à condition de bien étudier leur toxicité sur cellules humaines.

Cette toxicité hépatique de la TGZ a conduit à son retrait du marché en 2000 alors qu'elle était utilisée depuis 1997 pour ses propriétés antidiabétiques. La RGZ a été retirée du marché à l'automne 2010 pour des raisons de toxicité cardiaque. De même, la PGZ a été retirée du marché français car elle induirait un risque accru de développer un cancer de la vessie.

Il est donc nécessaire d'optimiser les propriétés antiprolifératives de la TGZ, tout en essayant d'en diminuer l'hépatotoxicité.

La Δ2TGZ, molécule analogue mais comportant une double liaison supplémentaire a été rapportée dans la littérature comme étant dépourvue d'activité PPARγ mais ayant des propriétés antiprolifératives comparables voire légèrement supérieures à la TGZ. Des dérivés de cette molécule ont été préparés par Chen et al. (J.-W. Huang, C.-W. Shiau, J. Yang, D.-S. Wang, H.-C. Chiu, C.-Y. Chen, C.-S. Chen, J. Med. Chem. 49 (2006) 4684-4689) qui ont ainsi obtenu des composés dont l'activité antiproliférative est de l'ordre du micromolaire. D'autres dérivés ont été décrits plus tard dans la littérature (J. Yang, S. Wei, D.-S. Wang, Y-C. Wang, S. K. Kulp, C.-S. Chen, J. Med. Chem. 51 (2008) 2100-2107 ; J.-H. Guh, W.-L. Chang, J. Yang, S.-L. Lee, S. Wei, D. Wang, S.K. Kulp, C.-S. Che, J. Med. Chem. 53 (2010) 2552-2561) et dans la demande internationale WO 2009/105621. Toutefois, ces composés présentent une certaine toxicité hépatique, et ne peuvent donc pas être utilisés.

Il est donc nécessaire de proposer de nouvelles molécules dérivées de thiazolidinediones présentant une faible toxicité hépatique, tout en ayant une activité antiproliférative supérieure à celle de la TGZ.

### Divulgation de l'invention

A cet effet, et conformément à la présente invention, il est proposé de nouveaux composés de formule (I) selon les revendications.

De tels composés présentent, à une concentration de 100 µM, une viabilité hépatocytaire supérieure à 60 %, de préférence supérieure à 80%.

### Mode(s) de réalisation de l'invention

Selon l'invention, il est proposé de nouveaux composés de formule (I) 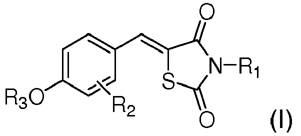 dans lesquels :
- R₃ est un groupe 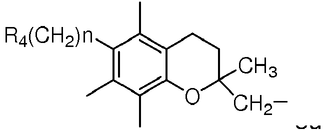 où n est supérieur à 1 et R₄ est choisi parmi le groupe comprenant les groupes H, OH, COOR, NRR', NHCOOR", NHM, R et R' étant choisis parmi H, un groupe alkyle et un groupe benzyle, R" étant un groupe choisi parmi un groupe alkyle et un groupe benzyle, et M étant un groupe fluorescent,
- R₁ est choisi parmi le groupe comprenant les groupes H, alkyle, benzyle, -(CH2)ₘ-CH=CH-φ, 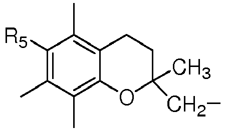 où m est supérieur ou égal à 1, R₅ est choisi parmi le groupe comprenant H, OH, COOR, OCOOR, (CH₂)ₚR₉, O(CH₂)ₚR₉, OCO(CH₂)ₚR₉, où p est supérieur ou égal à 1, de préférence supérieur ou égal à 2, R₉ étant choisi parmi le groupe comprenant les groupes H, OH, COOR, NRR', NHCOOR", NHM, R et R' étant choisis parmi H, un groupe alkyle et un groupe benzyle, R" étant un groupe choisi parmi un groupe alkyle et un groupe benzyle, et M étant un groupe fluorescent,
- R₂ est choisi parmi le groupe comprenant les groupes hydrogène, amino, méthoxy, éthoxy, nitro, phényl, di-alkyl, hydroxyl, lorsque R₁ est choisi parmi les groupes alkyle ou benzyle,
- R₂ est choisi parmi le groupe comprenant les groupes hydrogène, halo, amino, méthoxy, éthoxy, nitro, phényl, di-alkyl, di-halo, trifluorométhyl, hydroxyl, lorsque R₁ est choisi parmi le groupe comprenant l'hydrogène,
- (CH₂)ₘ-CH=CH-Φ et

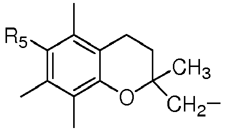

De préférence, R₂ est un hydrogène.

De préférence, n est compris entre 2 et 20, et préférentiellement compris entre 2 et 10 et plus préférentiellement entre 5 et 8.

Lorsque R₄ ou R₉ sont des groupes NRR', R₄ ou R₉ peuvent être un groupe NH₂, un groupe NHR (R étant par exemple un groupe butyle), ou un groupe NRR', (R et R' formant par exemple avec l'atome d'azote un résidu pipéridine).

La viabilité hépatocytaire correspond au pourcentage d'hépatocytes humains en suspension survivants après 90 minutes d'incubation avec les composés de l'invention présents à une concentration de 100 µM.

Avantageusement, les composés de l'invention présentent encore, à une concentration de 200 µM, une viabilité hépatocytaire supérieure à 60 %, plus préférentiellement supérieure à 80%.

Des composés préférés de l'invention sont tels que R₁ est H et R₄ est par exemple un groupe NRR', R et R' étant choisis parmi H, un groupe alkyle et un groupe benzyle, n étant compris entre 2 et 20, et de préférence entre 2 et 10, et préférentiellement entre 5 et 8. n peut être égal à 6.

Ainsi, le composé pour lequel R₃ est le groupe 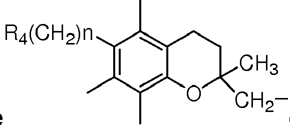 où n=1 et R₁, R₂ et R₄ sont des groupes hydrogène est exclu de l'invention.

Un composé particulièrement préféré est le composé de formule E :

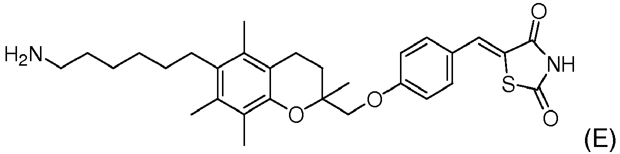

Il peut être présent sous la forme de sel afin d'augmenter sa solubilité sans gêner son activité.

Un composé de l'invention pour lequel R₄ est un groupe NHR est par exemple :

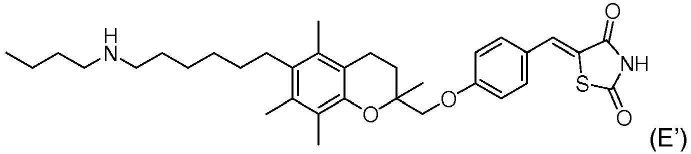

Un composé de l'invention pour lequel R₄ est un groupe NRR' est par exemple :

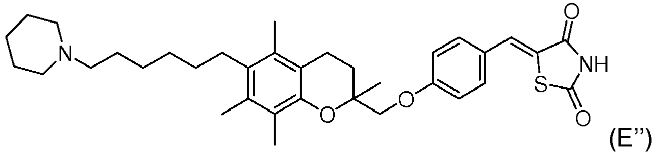

De tels composés pour lesquels R₄ est NHR sont obtenus en condensant les composés pour lesquels R₄ est NH₂ avec des aldéhydes variés, puis en réduisant l'imine obtenue avec par exemple du borohydrure de sodium.

De même, des composés pour lesquels R₄ est NRR' peuvent être obtenus en faisant réagir l'intermédiaire de synthèse bromé (molécule n°4 du schéma réactionnel 1) avec des amines secondaires, du type morpholine, pipéridine, pyrrolidine.

D'autres composés selon l'invention sont tels que R₁ est H et R₄ est un groupe NHCOOR", R" étant un groupe alkyle tel que méthyle, éthyle, propyle, isopropyle, butyle, isobutyle, *t*-butyle, ou un groupe benzyle.

Un composé particulièrement préféré est le composé de formule C :

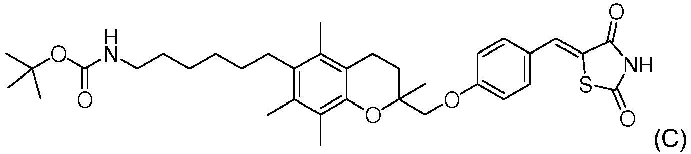

D'autres composés selon l'invention sont tels que R₄ est un groupe NHM, M étant un groupe fluorescent. De préférence, M est un groupe fluorescent issu d'un composé fluorophore choisi parmi le groupe comprenant la fluorescéine, le vert Oregon, la rhodamine, le rouge Texas, le bodipy, la cyanine.

D'autres composés selon l'invention sont tels que R₁ est choisi parmi le groupe comprenant les groupes alkyle, benzyle, -(CH₂)ₘ-CH=CH-Φ, 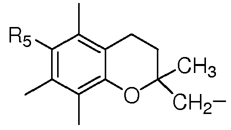 où m est supérieur ou égal à 1, R₅ est choisi parmi le groupe comprenant H, OH, COOH, COOR, OCOOR, (CH₂)ₚR₉, O(CH₂)ₚR₉, OCO(CH₂)ₚR₉, où p est supérieur ou égal à 1, de préférence supérieur ou égal à 2, R₉ étant choisi parmi le groupe comprenant les groupes H, OH, COOR, NRR', NHCOOR", NHM, R et R' étant choisis parmi H, un groupe alkyle et un groupe benzyle, R" étant un groupe choisi parmi un groupe alkyle et un groupe benzyle, et M étant un groupe où q est supérieur ou égal à fluorescent.

Parmi ces composés, des composés préférés selon l'invention sont tels que R₁ est un groupe alkyle, de préférence méthyle ou éthyle.

Parmi ces composés, des composés préférés sont tels que R₁ est un groupe alkyle, par exemple méthyle, et R₄ est un groupe NRR', R et R' étant choisis parmi H, un groupe alkyle et un groupe benzyle, n étant compris entre 2 et 20 et de préférence entre 2 et 10, et préférentiellement entre 5 et 8. peut être égal à 6.

Un composé particulièrement préféré est le composé de formule D :

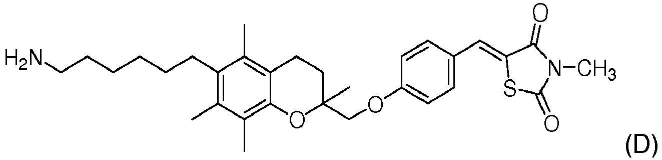

Il peut être présent sous la forme de sel.

De préférence, R est un groupe alkyle, tel que méthyle, éthyle, propyle, isopropyle, butyle, isobutyle, *t*-butyle.

Il est bien entendu que tous les isomères optiques et géométriques, notamment les formes énantiomères, ou diastéréoisomères et leurs mélanges, notamment les mélanges racémiques, ainsi que les sels d'addition avec des acides minéraux et organiques ou des bases minérales ou organiques des composés décrits ci-dessus appartiennent à la présente invention.

Les composés selon l'invention sont non toxiques sur les cultures primaires d'hépatocytes humains, contrairement à la TGZ ou aux dérivés connus. De plus, ils présentent une activité antiproliférative sur lignées de cellules cancéreuses humaines très supérieure à la TGZ et supérieure à celle des dérivés connus.

Plus particulièrement, les composés pour lesquels R₃ est le groupe 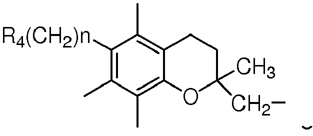 ne présentant pas d'oxygène lié au groupe chromane en position 6 et présentant à la place une chaine alkyle apparaissent d'une manière surprenante et inattendue comme des composés présentant une faible toxicité hépatique tout en conservant une activité antiproliférative.

D'autre part, il est également apparu d'une manière surprenante et inattendue que les composés pour lesquels l'azote de la thiazolidinedione est substitué par un groupe tel que défini ci-dessus, présentent une faible toxicité hépatique tout en conservant une activité antiproliférative.

Un procédé de synthèse des composés de formule (I) dans lesquels R₃ est le groupe 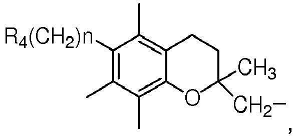 comprend la préparation d'une molécule intermédiaire de formule (II) 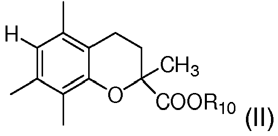 où R₁₀ est un groupe alkyle, tel que méthyle, éthyle.

Un procédé de synthèse de la molécule intermédiaire de formule II définie ci-dessus, comprend :
- une étape d'estérification du composé de formule III avec un alcool R₁₀OH 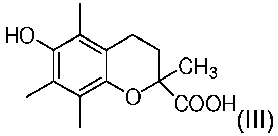
- une étape de fonctionnalisation de la fonction OH du composé III estérifié par un groupe partant,
- une étape d'hydrogénation sous pression en présence d'une base organique, permettant d'obtenir la molécule intermédiaire de formule (II).

Les deux dernières étapes sont basées sur un protocole décrit dans la littérature (E. Mahdavian, S. Sangsura, G. Landry, J. Eytina, B. A. Salvatore, Tetrahedron Lett. 50 (2009) 19-21).

De préférence, le groupe partant utilisé lors de l'étape de fonctionnalisation de la fonction OH est par exemple OSO₂CF₃, OSO₂PhCH₃ ou OSO₂CH₃, l'étape se déroulant en présence deTf₂O (anhydride triflique) ou de TosCl (Chlorure de tosyle) ou de MesCl (chlorure de mesyle) et de pyridine.

La base organique utilisée lors de l'étape d'hydrogénation est par exemple la triéthylamine.

Un autre procédé de synthèse de la molécule intermédiaire de formule II définie ci-dessus en une seule étape peut être proposé :

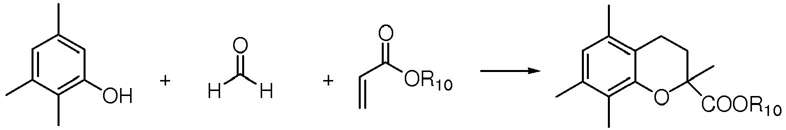

Les réactifs sont disponibles dans le commerce.

Ce procédé est basé sur un protocole décrit par E. A. Couladouros & al. Journal of Organic Chemistry 2007, 72, 6735-6741.

La présente invention concerne également une composition pharmaceutique, contenant à titre de principe actif une dose efficace d'un composé de formule (I) défini ci-dessus, ou un de ses sels d'addition avec des acides minéraux et organiques ou avec des bases minérales ou organiques pharmaceutiquement acceptables.

Le principe actif peut être mélangé à au moins un excipient pharmaceutiquement acceptable et/ou à au moins un autre principe actif.

Ces excipients sont connus de l'Homme du métier et seront adaptés à la forme pharmaceutique et au mode d'administration souhaité. Les compositions pharmaceutiques selon l'invention se présentent sous toutes les formes connues de l'Homme du métier, adaptées notamment pour une administration par voie orale, sublinguale, intramusculaire, intraveineuse, topique, locale, intranasale, transdermique ou rectale. Les compositions pharmaceutiques peuvent ainsi se présenter sous la forme de gélules, de comprimés, de granulés, de suppositoires, de préparations injectables, de crèmes, préparés selon des méthodes usuelles.

La présente invention concerne également un composé ou une composition pharmaceutique tels que définis ci-dessus, pour utilisation comme médicament.

La présente invention concerne également un composé ou une composition pharmaceutique tels que définis ci-dessus, pour utilisation comme médicament pour la prévention ou le traitement d'un cancer, et plus particulièrement le cancer du sein.

Les exemples suivants illustrent la présente invention sans toutefois en limiter la portée.

### PREPARATIONS

### Remarques générale :

Les solvants et les réactifs liquides ont été purifiés et séchés selon les procédures recommandées. L'eau utilisée pour le lavage des solutions organiques est de l'eau distillée. Les analyses par Chromatographie sur Couche Mince ont été réalisées suivant les procédures standard sur des plaques Kieselgel 60 F254 (Merck). Les composés ont été visualisés en utilisant une lampe UV (254 nm) et une solution de sulfate de cérium tétrahydrate et d'acide phosphomolybdique dans l'acide sulfurique aqueux à 10% comme agent de révélation. Une solution éthanolique de ninhydrine (0,3%) et d'acide acétique (3%) est aussi parfois utilisée. Les colonnes de chromatographie ont été réalisées avec de la silice Silica Gel SI 60 (63-200 µm) (Merck). Certaines (si indiqué) chromatographies ont été réalisées avec de la silice Silica Gel 60H (5-40 µm) (Merck) avec une machine « Axial® Modul Prep », travaillant à 8 bars, avec une colonne de 20 mm de diamètre. Les spectres RMN ¹H ont été enregistrés sur un spectromètre Bruker DPX250 (250 MHz). Les déplacements chimiques (δ) sont donnés en ppm par-rapport au pic résiduel du solvant. Les spectres de masse ont été obtenus sur un spectromètre VG-Platform Micromass-Waters (ESI+/- quad). Les analyses élémentaires ont été réalisées sur un appareil Thermofinnigan FlashEA 1112.

### Synthèse des composés de formule C, D et E:

La synthèse de ces dérivés s'effectue selon le schéma 1 réactionnel suivant : 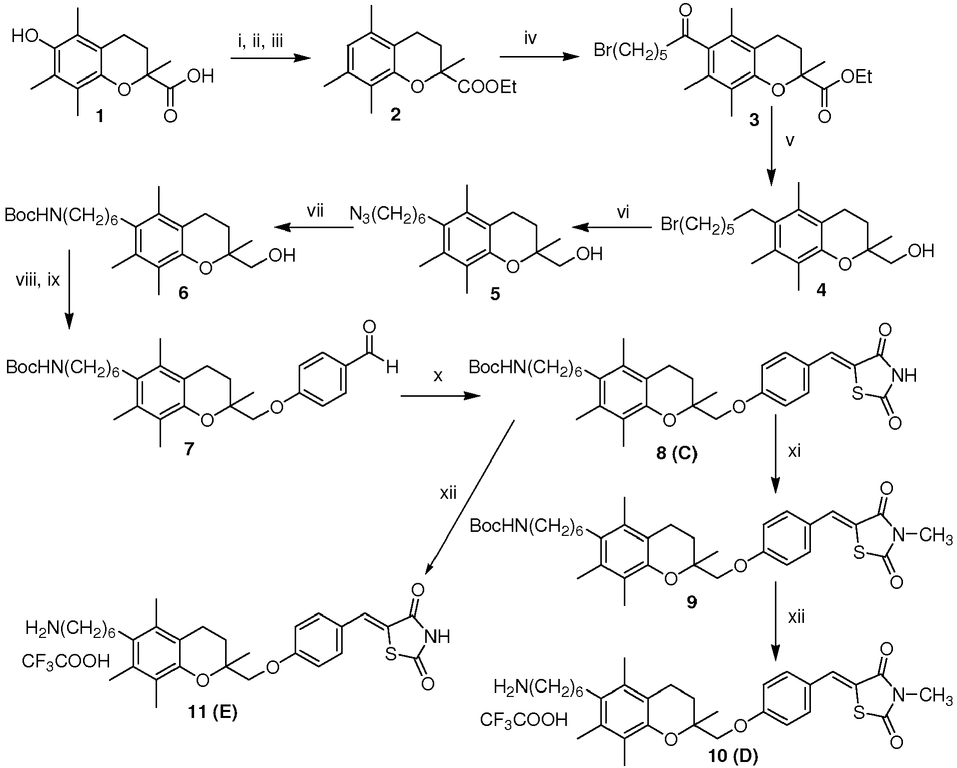 Schéma 1. Synthèse des composés C, D et E. Réactifs et conditions: i EtOH, cat. *p*-TSA, reflux 95%; ii Tf₂O, pyr., 99%; iii TEA, H₂ 70 psi, 10% Pd/C, 96%; iv Br(CH₂)₅COCl, AlCl₃ 70% ; v LiAlH₄, AlCl₃ 98%; vi NaN₃ 98% ; vii H₂ 1 atm., Pd/C, Boc₂O 70% ; viii Tf₂O, Pyr. quant. ; ix 4-hydroxybenzaldéhyde, K₂CO₃ 67%; x 2,4-thiazolidinedione, pipéridine, acide benzoïque, 81%; xi CH₃l, K₂CO₃ 65%; xii acide trifluoroacétique 54% (10, composé D), 53% (11, composé E).

### (±) (6-{2-[4-(2,4-Dioxo-thiazolidin-5-ylidenemethyl)-phénoxymethyl]-2,5,7,8-tétramethyl-chroman-6-yl}-hexyl)-carbamate de tert-butyle (composé C)

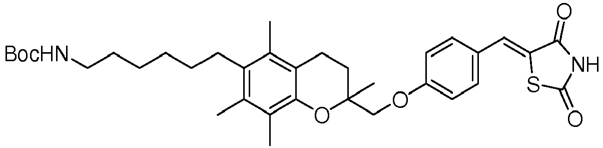

**Étape i** : à une solution d'acide (±) 6-hydroxy-2,5,7,8-tetramethylchroman-2-carboxylique (Trolox®) 1 (4,46 g ; 18,82 mmol) dans EtOH (175 mL) est ajouté l'acide *p*-toluène sulfonique (361 mg ; 1,90 mmol), et le mélange est chauffé à reflux pendant 96 h. La solution est concentrée sous vide, et le résidu est dissous dans AcOEt (150 mL). La solution résultante est lavée avec une solution aqueuse de NaHCO₃ 5% (2x50 mL) et une solution de NaCl saturée (2x50 mL). La phase organique est séchée (MgSO₄) et le solvant est évaporé. Le produit est séché soigneusement pour donner 4,98 g d'ester éthylique sous forme de cristaux blancs (17,90 mmol; rendement 95%).
**Étape ii :** à une solution de 500 mg (1, 80 mmol) de l'ester précédent dans le CH₂Cl₂ sec (10 mL) sous argon est ajoutée de la pyridine (872 µL, 10,78 mmol) et la solution est refroidie à 0°C. Ensuite, l'anhydride triflique (453 µL, 2,69 mmol) est ajouté goutte à goutte et la solution est agitée 1 h à température ambiante. La solution est diluée avec CH₂Cl₂ (20 mL) et lavée avec de l'eau (2 x 30 mL), séchée (MgSO₄) et concentrée à sec. Le résidu liquide est purifié par chromatographie sur colonne de silice (Hexane/AcOEt, 85:15) pour donner 735 mg (1,79 mmol ; rendement 99%) de triflate sous forme de liquide incolore.
**Étape iii :** on introduit d'abord dans le récipient d'un hydrogénateur de Parr du charbon palladié à 10% (3,00 g), pour éviter toute inflammation du solvant. Le charbon palladié utilisé est obtenu chez Fisher/ACROS (n° de catalogue: 19503-0500). Une solution du triflate précédent (4,96 g; 12,09 mmol) dans le THF (40 mL) est ajoutée au récipient, suivi par MeOH (80 mL), et TEA (7,4 mL; 53,22 mmol). La suspension est agitée sous une pression de 70 psi d'hydrogène pendant 20 h à température ambiante, puis filtrée sur celite® et la solution résultante est concentrée sous vide. Une chromatographie sur colonne de silice (Hexane/AcOEt, 90:10) donne 3,03 g (11,55 mmol; rendement 96%) de composé 2 ((±) 2,5,7,8-Tétramethyl-chroman-2-carboxylate d'éthyle) sous forme de liquide incolore qui pendant son stockage à 4 °C donne des cristaux blancs à bas point de fusion (environ 30 °C). RMN ¹H (CDCl₃) δ: 1,19 (t, *J* = 7,1 Hz, 3H, COOCH₂CH₃); 1,62 (s, 3H, CH₃); 1,82-1,95 (m, 1H, chromane 3-*Ha*Hb), 2,13 (s, 3H, ArCH₃); 2,16 (s, 3H, ArCH₃); 2,21 (s, 3H, ArCH₃); 2,38-2,69 (m, 3H, chromane 3-Ha*Hb* et 4-H₂); 4,14 (q, *J* = 7,1 Hz, 2H, COOCH₂CH₃); 6,58 (s, 1 H, Harom). ESI-MS (mode positif): m/z = 285,21 [M+Na]+. Anal. Calc. pour C₁₆H₂₂O₃ (262,4): C, 73,25; H, 8,45. Trouvé: C, 73,63; H, 8,20.
**Étape iv :** préparation du chlorure d'acide intermédiaire: une solution d'acide 1-Bromohexanoïque (2,02 g ; 10,3 mmol) et de chlorure de thionyle (2,2 ml ; 30,8 mmol) dans le CH₂Cl₂ sec (10 ml) est portée à reflux sous argon pendant 4 h. Le solvant est éliminé sous vide pendant plusieurs heures pour enlever tout l'excès de chlorure de thionyle. L'huile incolore obtenue est directement utilisée dans l'étape suivante sans purification supplémentaire. Réaction de Fidel-Craft: une suspension du chlorure d'acide précédent et de AlCl₃ (1,44 g ; 10,8 mmol) à 0°C dans le CH₂Cl₂ sec (3 mL) est ajoutée doucement à une solution du composé 2 dans le CH₂Cl₂ sec (4 mL). La réaction est agitée sous argon à 0°C pendant 1 h puis à température ambiante toute une nuit. Le réacteur est régulièrement purgé à l'argon pour éliminer le HCl. La réaction est précautionneusement arrêtée avec de l'eau distillée froide (6 mL) et une solution aqueuse d'acide chlorhydrique (2 mL). La phase organique est séparée et lavée avec une solution aqueuse de NaHCO₃ 5% (10 mL), séchée avec MgSO₄, filtrée et le solvant est éliminé sous vide pour donner un liquide jaune purifié ensuite par chromatographie sur colonne de silice (Hex:AcOEt 95:5) pour donner le composé 3 ((±) 6-(6-Bromo-hexanoyl)-2,5,7,8-tetraméthyl-chroman-2-carboxylate d'éthyle) sous forme d'un liquide incolore (690 mg ; 1,57 mmol, rendement 70%).
**Étape v :** à une suspension de LiAlH₄ (176 mg ; 4,64 mmol) dans Et₂O sec (6 ml) sous argon est ajoutée doucement une solution de AlCl₃ (413 mg ; 3,10 mmol). Cinq minutes plus tard, une solution de composé 3 (680 mg ; 1,55 mmol) et AlCl₃ (206 mg ; 1,55 mmol) dans Et₂O sec (12 ml) est ajoutée. Le mélange est agité sous argon à température ambiante pendant 2 h puis sont ajoutés précautionneusement de l'eau froide (24 ml) et une solution aqueuse de H₂SO₄ 6M. La phase organique est séparée et la phase aqueuse est extraite avec AcOEt (2 x 15 mL). Les phases organiques sont rassemblées puis lavées avec une solution aqueuse de Na₂CO₃ 2M (10 mL), séchées (MgSO₄), filtrées et le solvant évaporé sous vide pour donner le composé 4 ((±) [6-(6-Bromo-hexyl)-2,5,7,8-tetraméthyl-chroman-2-yl]-méthanol) sous forme de liquide incolore (592 mg ; 1,54 mmol ; rendement 98%) qui est directement utilisé pour l'étape suivante sans purification supplémentaire.
**Étape vi :** une suspension du composé 4 (592 mg; 1,54 mmol) et de NaN₃ (1,00 g; 15,4 mmol) dans le DMF sec (15 mL) est chauffée à 80°C pendant 6 h. Le mélange est refroidi à température ambiante, dilué avec AcOEt (60 mL) et lavé avec une solution saturée de NaCl (2 x 50 ml) et avec de l'eau (50 mL), séché (MgSO₄), filtré et le solvant est évaporé sous vide pour donner le composé 5 ([6-(6-Azido-hexyl)-2,5,7,8-tétramethyl-chroman-2-yl]-méthanol sous forme de liquide incolore (532 mg; 1,54 mmol; rendement 98%), qui est directement utilisé pour l'étape suivante sans purification supplémentaire.
**Étape vii :** une suspension du composé 5 (532 mg; 1,54 mmol), Pd/C (53 mg) et Boc₂O (403 mg; 1,85 mmol) dans MeOH sec (10 mL) est agitée à température ambiante sous atmosphère d'hydrogène pendant 12 h. Le mélange est filtré sur Célite® et le MeOH est évaporé sous vide. Le résidu est dissous dans AcOEt (20 mL) et la solution est lavée avec une solution aqueuse d'acide citrique 5% (2 x 15 mL), une solution aqueuse de NaHCO₃ 5% (2 x 15 mL) et de l'eau (20 mL), séchée (MgSO₄), filtrée et le solvant est évaporé sous vide pour donner un liquide jaune clair qui est purifié par chromatographie sur colonne de silice (Hex:EtOAc 8:2) pour donner le composé 6 ((±) [6-(2-Hydroxyméthyl-2,5,7,8-tetraméthyl-chroman-6-yl)-hexyl]-carbamate de tert-butyle) sous forme de liquide incolore (452 mg; 1,08 mmol, rendement 70%).
**Étape viii :** une solution de pyridine anhydre (430 µL ; 5,39 mmol) dans CH₂Cl₂ sec (5 mL) est refroidie à 0°C, puis est ajouté goutte à goutte l'anhydride trifluorométhanesulfonique (210 µL; 1,26 mmol) sous argon. Une solution du composé 6 (377 mg; 0,90 mmol) dans CH₂Cl₂ sec (5 mL) est ajoutée au mélange. La solution est agitée à 0°C pendant 20 min et le solvant est évaporé. L'excès de pyridine est co-évaporé deux fois avec du toluène et le résidu est dissous dans AcOEt (25 mL). La phase organique est lavée avec une solution aqueuse de NaHCO₃ 5% (2 x 15 mL) et une solution saturée de NaCl (20 mL), séchée (MgSO₄), filtrée et le solvant est évaporé pour donner le triflate désiré sous forme d'un liquide brun directement utilisé dans l'étape suivante.
**Étape ix :** à une solution du triflate précédent dans le DMF sec (3 mL) sont ajoutés le 4-hydroxybenzaldéhyde (164 mg ; 1,35 mmol) et K₂CO₃ (232 mg ; 1,68 mmol). Le mélange est agité sous argon à température ambiante pendant deux jours, puis de l'eau (8 mL) est ajoutée. La suspension est extraite avec AcOEt (2 x 20 mL). La phase organique est lavée avec une solution aqueuse de NaHCO₃ 5% (2 x 15 mL) et une solution saturée de NaCl (20 mL), séchée (MgSO₄), filtrée et le solvant est évaporé pour donner un liquide jaune ensuite purifié par chromatographie sur colonne de silice (Hexane:AcOEt 9:1) pour donner le composé 7 ((±) {6-[2-(4-Formyl-phénoxymethyl)-2,5,7,8-tétramethyl-chroman-6-yl]-hexyl}-carbamate de tert-butyle) sous forme de solide blanc (316 mg ; 0,60 mmol ; rendement 67%).
**Étape x:** une solution du composé 7 (68 mg ; 0,13 mmol), 2,4-thiazolidinedione (31 mg ; 0,26 mmol), pipéridine (70 µL; 0,07 mmol) et d'acide benzoïque (80 mg ; 0,07 mmol) dans le toluène sec (3 mL) est portée à reflux pendant une nuit sous argon. Le mélange est refroidi à température ambiante, dilué avec AcOEt (10 mL), lavé avec une solution de HCl 1N (5 mL), une solution de NaHCO₃ 5% (5 mL) et une solution saturée de NaCl (5 mL), séché (MgSO₄), filtré puis le solvant est évaporé pour donner un liquide jaune qui est purifié par précipitation dans MeOH froid pour donner le composé de formule C (66 mg ; 0,10 mmol, rendement 81%) sous forme de solide légèrement jaune. RMN ¹H (CDCl₃) δ: 1,43 (s, 3H, CH₃); 1,36-1,44 (m, 4H, linker-CH₂); 1,45 (s, 9H, -*t*Bu); 1,55-1,69 (m, 4H, linker-CH₂); 1,82-1,98 (m, 1H, 3-*Ha*Hb chromane); 2,10-2,14 (m, 1H, 3-Ha*Hb* chromane); 2,09 (s, 3H, ArCH₃); 2,15 (s, 3H, ArCH₃); 2,19 (s, 3H, ArCH₃); 2,52-2,74 (m, 4H, 4-H₂ chromane, CH₂ linker); 3,06-3,18 (m, 2H, CH₂ linker); 3,96; 4,06 (système AB, *J* = 9,4 Hz, 2H, CH₂O); 4,50 (sl, 1H, NHBoc); 7,01 (d, *J* = 9,0 Hz, 2H, Hₐᵣₒₘ); 7,43 (d, *J* = 9,0 Hz, 2H, Hₐᵣₒₘ); 7,80 (s, 1H, ArCH=); 8,41 (sl, 1H, NH). ESI-MS (mode positif): m/z = 645.29 [M+Na]⁺. Anal. Calc. pour C₃₅H₄₆N₂O₆S, 1/3 H₂O (628.8): C, 66,85; H, 7,48; N, 4,45. Trouvé: C, 66,83; H, 7,27; N, 4,47.

### (±) Trifluoroacétate de 5-{4-[6-(6-amino-hexyl)-2,5,7,8-tetraméthyl-chroman-2-ylméthoxy]-benzylidene}-3-méthyl-thiazolidine-2,4-dione (composé D)

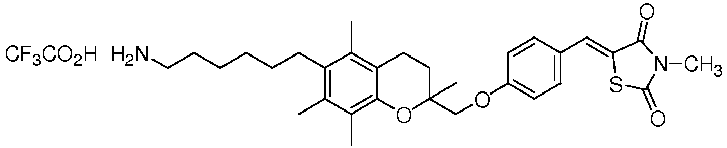

**Étape xi :** à une solution du composé C (230 mg; 0,37 mmol) dans le DMF sec (3 ml) sont ajoutés K₂CO₃ (66 mg ; 0,48 mmol) et l'iodure de méthyle (28 µL; 0,44 mmol). Le mélange est agité sous argon à 80°C pendant 3 h. Il est alors dilué avec AcOEt (30 mL) et la solution est lavée avec une solution saturée de NaCl (20 mL) et de l'eau (20 mL), séchée (MgSO₄), filtrée et le solvant est évaporé sous vide pour donner un liquide jaune qui est purifié par précipitation dans MeOH froid pour donner le composé 9 (±) ((6-{2,5,7,8-Tétramethyl-2-[4-(3-méthyl-2,4-dioxo-thiazolidin-5-ylideneméthyl)-phénoxyméthyl]-chroman-6-yl}-hexyl)-carbamate de tert-butyle) sous forme de solide incolore (154 mg ; 0,24 mmol, rendement 65%).
**Étape xii :** une solution du composé 9 (30 mg; 48,2 µmol) dans CH₂Cl₂ sec (2 mL) est refroidie à 0°C, puis est ajouté l'acide trifluoroacétique (1 mL). La réaction est portée à température ambiante et agitée pendant 3 h. Le solvant est évaporé et l'excès d'acide est co-évaporé avec MeOH (5 fois). Le solide obtenu est lavé avec Et₂O pour donner le composé de formule D (17 mg ; 26,1 µmol, rendement 54%) sous forme d'un solide incolore. RMN ¹H (CDCl₃) δ: 1,42 (s, 3H, CH₃); 1,28-1,57 (m, 6H, CH₂ linker); 1,57-1,80 (m, 2H, CH₂ linker); 1,82-1,98 (m, 1H, 3-*Ha*Hb chromane); 2,08-2,17 (m, 1H, 3-Ha*Hb* chromane); 2,08 (s, 3H, ArCH₃); 2,13 (s, 3H, ArCH₃); 2,17 (s, 3H, ArCH₃); 2,44-2,75 (m, 4H, 4-H₂ chromane, CH₂ linker); 2,80-3,10 (m, 2H, CH₂ linker); 3,23 (s, 3H, NCH₃); 3,95 , 4,05 (système AB, *J* = 9,4 Hz, 2H, CH₂O); 6,99 (d, *J* = 8,0 Hz, 2H, Hₐᵣₒₘ); 7,43 (d, *J* = 8,0 Hz, 2H, Hₐᵣₒₘ); 7,85 (s, 1H, ArCH=); 7,85-8,02 (sl, 2H, NH₂). ESI-MS (mode positif): m/z = 537,29 [M+H]+. Anal. Calc. pour C₃₃H₄₁F₃N₂O₆S (536,2): C, 60,91; H, 6,35; N, 4,30. Trouvé: C, 60,44; H, 6,31; N, 4,20.

### (±) Trifluoroacétate de 5-{4-[6-(6-Amino-hexyl)-2,5,7,8-tétramethyl-chroman-2-ylméthoxy]-benzylidene}-thiazolidine-2,4-dione (composé E)

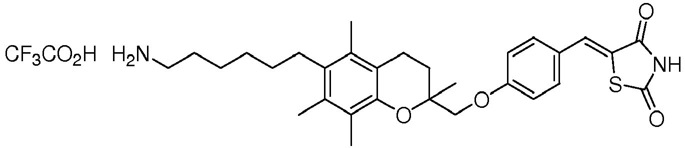

**Étape xii :** de même que précédemment, une solution du composé C (44 mg; 70,6 µmol) dans CH₂Cl₂ sec (2 mL) est refroidie à 0°C, puis l'acide trifluoroacétique (1 mL) est ajouté. La réaction est portée à température ambiante et agitée pendant 3 h. Le solvant est évaporé et l'excès d'acide est co-évaporé avec MeOH (5 fois). Le solide jaune obtenu est lavé avec Et₂O pour donner le composé de formule E (24 mg ; 37,7 µmol ; rendement 53%) sous forme d'un solide jaune clair. RMN ¹H (MeOD) δ: 1,43 (s, 3H, CH₃); 1,44-1,57 (m, 6H, CH₂ linker); 1,60-1,79 (m, 2H, CH₂ linker); 1,85-2,00 (m, 1H, 3-*Ha*Hb chromane); 1,90 (s, 3H, ArCH₃); 2,06-2,18 (m, 1H, 3-Ha*Hb* chromane); 2,18 (s, 3H, ArCH₃); 2,19 (s, 3H, ArCH₃); 2,59-2,76 (m, 4H, 4-H₂ chromane, CH₂ linker); 2,95 (t, *J* = 7,3 Hz, 2H, CH₂ linker); 4,09 (s, 2H, CH₂O); 7,08 (d, *J* = 8,8 Hz, 2H, Harom); 7,50 (d, *J* = 8,8 Hz, 2H, Harom); 7,76 (s, 1 H, ArCH=). ESI-MS (mode positif): m/z = 523,26 [M+H]⁺. Anal. Calc. pour C₃₂H₃₉F₃N₂O₆S, H₂O (654,74): C, 58,70; H, 6,31; N, 4,28. Trouvé: C, 59.08; H, 6,18; N, 4,18.

### EXEMPLES

### TOXICITE HEPATIQUE

La viabilité hépatocytaire a été mesurée pour différents composés selon l'invention avec des hépatocytes humains (culture primaire) cryopréservés mis en suspension et répartis dans des plaques à 96 puits à une densité de 0.1 x 10⁶ cellules par puits, dans un milieu de culture de 50 µL par puits (DMEM+gentamycine (50 mg/L) + insuline (4 mg/L) + dexamethasone (1 µM)) en atmosphère humidifiée CO₂/air (5%/95%) à 37°C. Après 30 minutes pour atteindre l'équilibre, 50 µL de milieu de culture contenant les composés testés (dissous dans le DMSO) ou de milieu contenant uniquement du DMSO (0,1% concentration finale) ont été ajoutés sous agitation à 900 tr/minute.

La cytotoxicité est évaluée par un test MTT (Thiazolyl Blue Tetrazolium Bromide). Les composés testés ou le DMSO sont laissés incuber 90 minutes, puis 10 µL de MTT à 10 mg/mL sont ajoutés par puits pendant 30 min. Ensuite, les plaques sont centrifugées pendant 10 minutes à 2000 g, le MTT est éliminé et du DMSO est ajouté à raison de 100 µL par puits. Les plaques sont secouées doucement puis l'absorbance est mesurée à 595 nm au moyen d'un spectrophotomètre lecteur de microplaques. La viabilité hépatocytaire mesurée ici correspond au pourcentage d'hépatocytes survivants après 90 minutes d'incubation avec les composés testés à une concentration de 100 µM, par rapport à des cellules non traitées.

Les mêmes tests sont réalisés pour une concentration en composés à 200 µM.

A titre comparatif, la viabilité hépatocytaire est également mesurée pour la troglitazone (composé K) 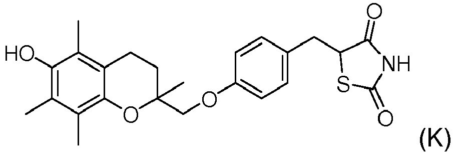 et un composé de formule L décrit par Chen et coll. dans la demande de brevet WO 2009/105621 :

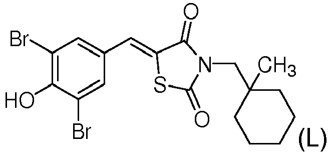

Les tests ont été réalisés en deux campagnes distinctes. Dans les deux cas, la troglitazone (K) a été utilisée comme molécule de référence.

Les résultats des deux campagnes sont indiqués dans le Tableau I ci-dessous :

**Tableau I**

| Composé | Viabilité hépatocytaire % (100 µM) | Viabilité hépatocytaire % (200 µM) |
|---|---|---|
| C | 85 | 80 |
| D | 83 | 83 |
| E | 83 | 80 |
| K (comp.) | 79 | 30 |
| L (comp.) | 42 | 30 |

Les résultats ci-dessus montrent que les composés selon l'invention présentent à 100 µM et à 200 µM une viabilité hépatocytaire supérieure à 80%, et même pour C égale à 85%, alors que le composé comparatif décrit par Chen et coll. présente une viabilité hépatocytaire de 42% seulement à 100 µM et de 30% à 200 µM, et la troglitazone présente une viabilité hépatocytaire de 30% seulement à 200 µM.

### TEST SUR DES SOURIS

Un test de toxicité a été réalisé sur des souris pour les molécules D et E. Ces molécules ne différent donc que par le groupe méthyle substitué sur la thiazolidinedione. A titre comparatif, le test a également été réalisé en utilisant la Δ2TGZ. Ces analyses ont été menées sur des souris femelles NMRI de 20-25 g. Des groupes de 3 souris ont été constitués : un lot témoin non injecté, un lot recevant le solvant des molécules à tester (mélange eau PPI, polyéthylène glycol 400, éthanol absolu dans les proportions 5/3/2), trois autres lots recevant les molécules à tester. Les composés ont été injectés dans la veine caudale des souris préalablement anesthésiées. Les animaux ont été pesés avant puis 24h et 48h après l'injection intraveineuse des produits aux différentes doses testées puis ils ont aussi été pesés 3 fois par semaine pendant 2 semaines. Le comportement de l'ensemble des souris a été observé avant, puis 1h, 2h et 3h après l'injection intraveineuse puis quotidiennement pendant 2 semaines. Les composés ont été testés d'abord à la limite de leur solubilité. En fonction des résultats obtenus (mortalité, perte de poids, changement de comportement), des doses plus faibles ont été utilisées pour déterminer de façon précise la dose maximale tolérée. Lorsque moins de 50% de mortalité étaient observés ou moins de 50% d'animaux présentaient une perte de poids supérieure à 25% ou moins de 50% d'animaux présentaient des modifications comportementales la dose de composé était considérée comme non toxique (DMT).

La dose maximum tolérée de D est de 65,5 mg/kg, et de 34 mg/kg pour E. Le résultat pour la Δ2TGZ est inférieur à 34 mg/kg.

Ceci montre l'effet protecteur lié à la présence du groupe méthyle sur la thiazolidinedione puisque D et E ne diffèrent que par ce groupe.

### ACTIVITE ANTIPROLIFERATIVE

L'activité antiproliférative a été mesurée pour différents composés selon l'invention par analyse de la cytotoxicité à 10⁻⁵M sur diverses lignées cellulaires cancéreuses dans du DMSO en triplicate. Pour certaines molécules et pour certaines lignées, le même protocole a été utilisé pour déterminer les valeurs d'IC50 (concentration en µM nécessaire pour inhiber de moitié la prolifération des cellules), en effectuant les tests de cytotoxicité à des concentrations de 0,005 µM à 100 µM en duplicate. Le protocole est le suivant. Les cellules de la lignée envisagée sont ensemencées dans le milieu approprié (voir tableau II) additionné de L-Glutamine, de 10% (v/v) de sérum de veau foetal, 100 UI de pénicilline, 100 µg/mL de streptomycine et 1.5 µg/mL de fungizone, et maintenues sous 5% de CO₂ à 37°C. Des plaques à 96 puits sont ensemencées avec le nombre requis de cellules par puits (voir tableau II) dans 200 µL de milieu. Vingt-quatre heures plus tard, les composés chimiques à étudier dissous dans le DMSO sont additionnés et les milieux sont maintenus pendant 72 h dans ces conditions avec une concentration finale en DMSO fixe (1%). Les contrôles ont reçu un volume égal de DMSO. Le nombre de cellules viables est mesuré à 490 nm avec le réactif MTS (Promega, Madison, WI). Les IC50 sont calculées comme les concentrations de composés induisant une inhibition de la prolifération cellulaire de 50%.

**Tableau II**

| Lignée cellulaire | Nombre de cellules par puits | Milieu de culture |
|---|---|---|
| MCF7 | 1900 | RPMI |
| HCT116 | 1800 | RPMI |
| MDA231 | 1300 | RPMI |
| MDA435 | 1000 | RPMI |
| Mia-PaCa | 1200 | DMEM |
| HepG2 | 8000 | DMEM |
| PC3 | 2500 | RPMI |
| LNCaP | 5000 | RPMI |

À titre comparatif, les essais ont été également réalisés avec la troglitazone (composé K) et le composé de formule L décrit par Chen et coll. dans la demande de brevet WO 2009/105621 défini ci-dessus.

Les tableaux III et IV ci-dessous indiquent le pourcentage d'inhibition de la croissance cellulaire, c'est-à-dire le pourcentage de cellules mortes après traitement avec le composé dosé à 10⁻⁵M. Les tests ont été réalisés en triplicate.

**Tableau III**

| Composé | MCF7 | HCT116 | MDA231 | MDA435 |
|---|---|---|---|---|
| C | 24 | 55 | 51 | 39 |
| D | 101 | 101 | 101 | 101 |
| E | 97 | 93 | 92 | 65 |
| K (comp.) | 3 | 9 | 12 | 12 |
| L (comp.) | 76 | 87 | 53 | 69 |

**Tableau IV**

| Composé | Mia-PaCa | HepG2 | PC3 | LNCaP |
|---|---|---|---|---|
| C | 30 | 77 | 26 | 68 |
| D | 103 | 100 | 92 | 88 |
| E | 94 | 87 | 40 | 53 |
| K (comp.) | 2 | 14 | 14 | 17 |
| L (comp.) | 65 | 74 | 47 | 81 |

Les tableaux III et IV ci-dessus montrent que les composés selon l'invention ont une activité antiproliférative très supérieure à celle de la troglitazone. Par ailleurs, les résultats permettent de confirmer que le composé L décrit par Chen et coll. est assez actif sur toutes les lignées, mais un peu moins que les plus actifs des composés selon l'invention.

À titre indicatif, les IC50 (µM) mesurées de quelques-uns des composés précédents sur quelques lignées cancéreuses sont indiquées dans le tableau V.

**Tableau V**

| Composé | MCF7 | MDA231 | Mia-PaCa | HepG2 | LNCaP |
|---|---|---|---|---|---|
| C | 9,6 | 2,1 | 8,8 | 2,4 | 6,4 |
| D | 6,9 | 4,6 | 3,2 | 7,9 | 6,5 |
| E | 6,4 | 4,7 | 3,9 | 3,1 | 5,1 |
| L (comp.) | 9,3 | 5,7 | 8,1 | 8,8 | 1,8 |

Les valeurs indiquées sont les moyennes de deux valeurs indépendantes.

Ainsi, ces résultats montrent que, contrairement aux composés développés dans l'art antérieur, les nouveaux composés selon la présente invention ne sont pas toxiques sur les hépatocytes, tout en conservant des bonnes propriétés d'activité antiproliférative.

## Revendications

1. Composé de formule (I) 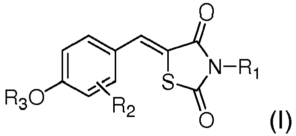 **caractérisé en ce qu'**il présente, à une concentration de 100 µM, une viabilité hépatocytaire supérieure à 60 %, et **en ce que**:
- R₃ est un groupe 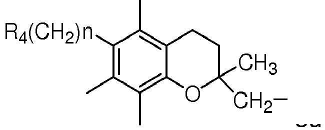 où n est supérieur à 1 et R₄ est choisi parmi le groupe comprenant les groupes H, OH, COOR, NRR', NHCOOR", NHM, R et R' étant choisis parmi H, un groupe alkyle et un groupe benzyle, R" étant un groupe choisi parmi un groupe alkyle et un groupe benzyle, et M étant un groupe fluorescent,
- R₁ est choisi parmi le groupe comprenant les groupes H, alkyle, benzyle, -(CH₂)ₘ-CH=CH-φ, 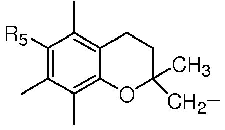 où m est supérieur ou égal à 1, R₅ est choisi parmi le groupe comprenant H, OH, COOR, OCOOR, (CH₂)ₚR₉, O(CH₂)ₚR₉, OCO(CH₂)ₚR₉, où p est supérieur ou égal à 1, R₉ étant choisi parmi le groupe comprenant les groupes H, OH, COOR, NRR', NHCOOR", NHM, R et R' étant choisis parmi H, un groupe alkyle et un groupe benzyle, R" étant un groupe choisi parmi un groupe alkyle et un groupe benzyle, et M étant un groupe fluorescent,
- R₂ est choisi parmi le groupe comprenant les groupes hydrogène, amino, méthoxy, éthoxy, nitro, phényl, di-alkyl, hydroxyl, lorsque R₁ est choisi parmi les groupes alkyle ou benzyle,
- R₂ est choisi parmi le groupe comprenant les groupes hydrogène, halo, amino, méthoxy, éthoxy, nitro, phényl, di-alkyl, di-halo, trifluorométhyl, hydroxyl, lorsque R₁ est choisi parmi le groupe comprenant l'hydrogène, -(CH₂)ₘ-CH=CH-Φ et

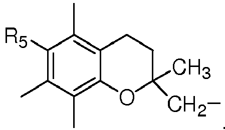

2. Composé selon la revendication 1, **caractérisé en ce que** R₁ est H et R₄ est un groupe NRR', R et R' étant choisis parmi H, un groupe alkyle et un groupe benzyle.

3. Composé selon la revendication 2 de formule E:

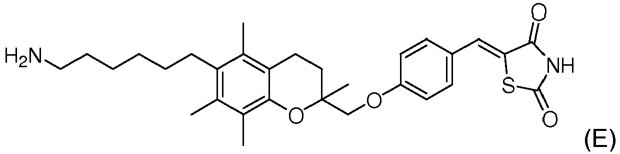

4. Composé selon la revendication 1, **caractérisé en ce que** R₁ est H et R₄ est un groupe NHCOOR", R" étant un groupe choisi parmi un groupe alkyle et un groupe benzyle.

5. Composé selon la revendication 4 de formule C :

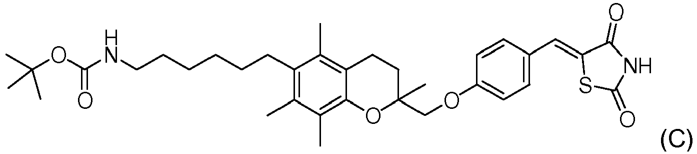

6. Composé selon la revendication 1, **caractérisé en ce que** R₁ est un groupe alkyle.

7. Composé selon la revendication 6, **caractérisé en ce que** R₁ est un groupe alkyle et R₈ est un groupe NRR', R et R' étant choisis parmi H, un groupe alkyle et un groupe benzyle.

8. Composé selon la revendication 7 de formule D :

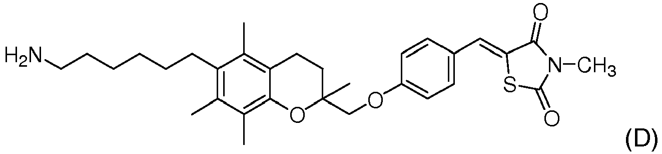

9. Composition pharmaceutique, **caractérisée en ce qu'**elle contient à titre de principe actif au moins un composé de formule (I) selon l'une quelconque des revendications 1 à 8, ou un de ses sels d'addition avec des acides minéraux et organiques ou avec des bases minérales ou organiques pharmaceutiquement acceptables.

10. Composition pharmaceutique selon la revendication 9, **caractérisée en ce que** le principe actif est mélangé à au moins l'un des composés choisis parmi au moins un excipient pharmaceutiquement acceptable et au moins un autre principe actif.

11. Composé selon l'une quelconque des revendications 1 à 8 pour utilisation comme médicament.

12. Composé pour utilisation selon la revendication 11, pour la prévention ou le traitement d'un cancer.

13. Composé pour utilisation selon la revendication 12 pour la prévention ou le traitement d'un cancer du sein.

14. Composition pharmaceutique selon l'une quelconque des revendications 9 et 10, pour utilisation pour la prévention ou le traitement d'un cancer.

15. Composition pharmaceutique pour utilisation selon la revendication 14 pour la prévention ou le traitement d'un cancer du sein.

## Patentansprüche

1. Verbindung der Formel (I) 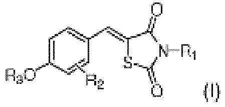 **dadurch gekennzeichnet, dass** sie aufweist, in einer Konzentration von 100 µM, eine hepatozytäre Viabilität über 60 %, und dadurch, dass:
- R₃ eine Gruppe 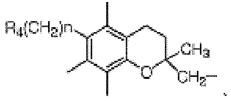 ist, wobei n größer als 1 ist und R₄ aus der Gruppe ausgewählt ist, welche die Gruppen H, OH, COOR, NRR', NHCOOR", NHM umfasst, wobei R und R' aus H, einer Alkylgruppe und einer Benzylgruppe ausgewählt sind, wobei R" eine Gruppe ist, die aus einer Alkylgruppe und einer Benzylgruppe ausgewählt ist und M eine fluoreszierende Gruppe ist,
- R¹ aus der Gruppe ausgewählt ist, die die H-, Alkyl-, Benzylgruppen, -(CH₂)ₘ-CH=CH-Φ 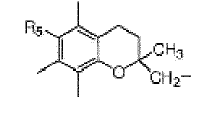 umfasst, wobei m größer oder gleich 1 ist, R₅ aus der Gruppe ausgewählt ist, die H, OH, COOR, OCOOR, (CH₂)ₚR₉, O(CH₂)ₚR₉, OCO(CH₂)ₚR₉ umfasst, wobei p größer oder gleich 1 ist, wobei R₉ aus der Gruppe ausgewählt ist, die die Gruppen H, OH, COOR, NRR', NHCOOR", NHM umfasst, wobei R und R' aus H, einer Alkylgruppe und einer Benzylgruppe ausgewählt sind, wobei R" eine Gruppe ist, die aus einer Alkylgruppe und einer Benzylgruppe ausgewählt ist, und M eine fluoreszierende Gruppe ist,
- R₂ aus der Gruppe ausgewählt ist, die die Wasserstoff-, Amino-, Methoxy-, Ethoxy-, Nitro-, Phenyl-, Dialkyl-, Hydroxylgruppen umfasst, wenn R₁ aus den Alkyl- oder Benzylgruppen ausgewählt ist,
- R₂ aus der Gruppe ausgewählt ist, die die Wasserstoff-, Halo-, Amino-, Methoxy-, Ethoxy-, Nitro-, Phenyl-, Dialkyl-, Dihalo-, Trifluormethyl-, Hydroxylgruppen umfasst, wenn R1 aus der Gruppe ausgewählt ist, die Wasserstoff, -(CH₂)ₘ-CH=CH-Φ und 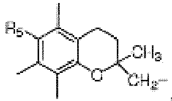 umfasst.

2. Verbindung nach Anspruch 1, **dadurch gekennzeichnet, dass** R₁ H ist und R₄ eine Gruppe NRR' ist, wobei R und R' aus H, einer Alkylgruppe und einer Benzylgruppe ausgewählt sind.

3. Verbindung nach Anspruch 2 der Formel E:

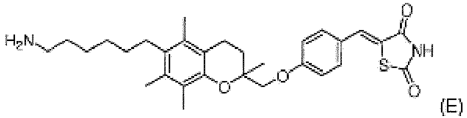

4. Verbindung nach Anspruch 1, **dadurch gekennzeichnet, dass** R₁ H ist und R₄ eine Gruppe NHCOOR" ist, wobei R" eine Gruppe ist, die aus einer Alkylgruppe und einer Benzylgruppe ausgewählt ist.

5. Verbindung nach Anspruch 4 der Formel C:

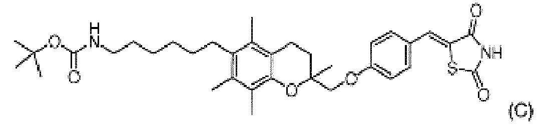

6. Verbindung nach Anspruch 1, **dadurch gekennzeichnet, dass** R₁ eine Alkylgruppe ist.

7. Verbindung nach Anspruch 6, **dadurch gekennzeichnet, dass** R₁ eine Alkylgruppe ist und R₈ eine Gruppe NRR' ist, wobei R und R' aus H, einer Alkylgruppe und einer Benzylgruppe ausgewählt sind.

8. Verbindung nach Anspruch 7 der Formel D:

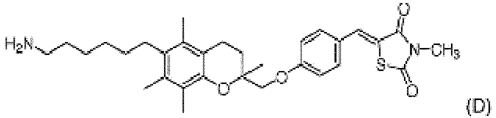

9. Pharmazeutische Zusammensetzung, **dadurch gekennzeichnet, dass** sie als Wirkstoff mindestens eine Verbindung der Formel (I) nach einem der Ansprüche 1 bis 8 enthält oder eines ihrer Additionssalze mit mineralischen und organischen Säuren oder mit mineralischen oder organischen Basen, die pharmazeutisch akzeptabel sind.

10. Pharmazeutische Zusammensetzung nach Anspruch 9, **dadurch gekennzeichnet, dass** der Wirkstoff mit mindestens einer der Verbindungen gemischt ist, die aus mindestens einem pharmazeutisch akzeptablen Hilfsstoff und mindestens einem anderen Wirkstoff ausgewählt sind.

11. Verbindung nach einem der Ansprüche 1 bis 8 für die Verwendung als Arzneimittel.

12. Verbindung für die Verwendung nach Anspruch 11 zur Vorbeugung oder zur Behandlung einer Krebserkrankung.

13. Verbindung für die Verwendung nach Anspruch 12 zur Vorbeugung oder zur Behandlung einer Krebserkrankung der Brust.

14. Pharmazeutische Zusammensetzung nach einem der Ansprüche 9 und 10 für die Verwendung zur Vorbeugung oder zur Behandlung einer Krebserkrankung.

15. Pharmazeutische Zusammensetzung nach Anspruch 14 zur Vorbeugung oder zur Behandlung einer Krebserkrankung der Brust.

## Claims

1. A compound of formula (I) 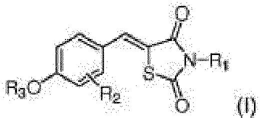 **characterized in that**, at a concentration of 100 µM, it has hepatocyte viability higher than 60 %, and **in that**
- R₃ is a group: 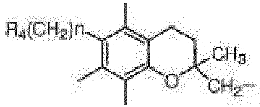 where: n is higher than 1 and R₄ is selected from the group comprising the groups H, OH, COOR, NRR' , NHCOOR" , NHM, R and R' being selected from among H, an alkyl group and benzyl group, R" being a group selected from among an alkyl group and benzyl group, and M being a fluorescent group;
- R₁ is selected from the group comprising the groups H, alkyl, benzyl,-(CH2)m-CH=CH-Φ, 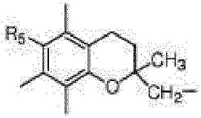 where m equals 1 or higher, R₅ is selected from the group comprising H, OH, COOR, OCOOR, (CH₂)ₚR₉, O(CH₂)ₚR₉, OCO(CH₂)ₚR₉ where p equals 1 or higher, R₉ being selected from the group comprising the groups H, OH, COOR, NRR' , NHCOOR" , NHM, R and R' being selected from among H, an alkyl group and benzyl group, R" being a group selected from among an alkyl group and benzyl group, and M being a fluorescent group,
- R₂ is selected among the group comprising the groups hydrogen, amino, methoxy, ethoxy, nitro, phenyl, di-alkyl, hydroxyl, when R₁ is selected from among the alkyl or benzyl groups,
- R₂ is selected among the group comprising the groups hydrogen, halo, amino, methoxy, ethoxy, nitro, phenyl, di-alkyl, di-halo, trifluoromethyl, hydroxyl when R₁ is selected from among the group comprising hydrogen, -(CH₂)ₘ-CH=CH-Φ and

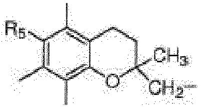

2. The compound according to claim 1, **characterized in that** R₁ is H and R₄ is a NRR' group, R and R' being selected from among H, an alkyl group and benzyl group.

3. The compound according to claim 2 of formula E:

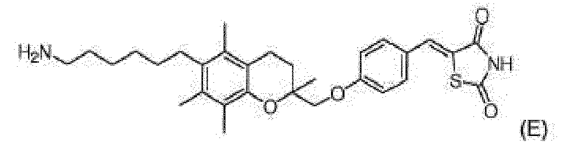

4. The compound according to claim 1, **characterized in that** R₁ is H and R₄ is a NHCOOR" group, R" being a group selected from among an alkyl group and benzyl group.

5. The compound according to claim 4 of formula C:

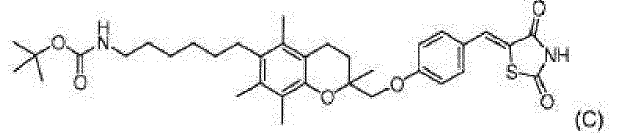

6. The compound according to claim 1, **characterized in that** R₁ is an alkyl group.

7. The compound according to claim 6, **characterized in that** R₁ is an alkyl group and R₈ an NRR' group, R and R' being selected from among H, an alkyl group and benzyl group.

8. The compound according to claim 7 of formula D:

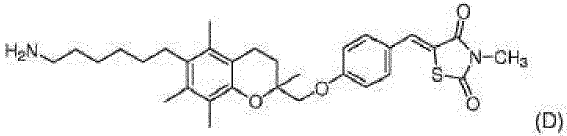

9. A pharmaceutical composition, **characterized in that** as active ingredient it contains at least one compound of formula (I) according to any of claims 1 to 8, or one of the addition salts thereof with pharmaceutically acceptable mineral and organic acids or mineral or organic bases.

10. The pharmaceutical composition according to claim 9, **characterized in that** the active ingredient is mixed with at least one of the compounds selected from among at least one pharmaceutically acceptable excipient and at least one other active ingredient.

11. The compound according to any of claims 1 to 8 for use as medicinal product.

12. The compound for use according to claim 11, in the prevention or treatment of cancer.

13. The compound for use according to claim 12 in the prevention or treatment of breast cancer.

14. The pharmaceutical composition according to any of claims 9 and 10 for use in the prevention or treatment of cancer.

15. The pharmaceutical composition for use according to claim 14 in the prevention or treatment of breast cancer.
